# EUROPEAN PATENT APPLICATION

(11) **EP 0 914 778 A1**
(43) Date of publication of application: **12.05.1999**
(21) Application number: 98306507.9
(22) Date of filing: 14.08.1998
(51) Int. Cl.: A23K 1/16, A23K 1/18, A23L 1/03, A61K 35/74

(54) **Oral administration of bacteria at a concentration which produces cell-mediated immunity and weight in certain animals**

(30) Priority: 15.08.1997 US 912026
(71) Applicant: Consolidated Nutrition, L.C., Omaha, Nebraska 68103-2048 (US); VETOQUINOL S.A., 70204 Lure Cedex (FR)
(72) Inventor: Edwards, Bobby G., Land-O-Lakes, Florida 34639-5527 (US); Pollmann, Steven D., Omaha, Nebraska 68135 (US); Van Kampen, Kent R., Ogden, Utah 84405 (US); Hottell, John D., Decatur, Indiana 46733 (US); Ward, Terry L., Fort Wayne, Indiana 46804 (US); Asche, Gary L., Fort Wayne, Indiana 46804 (US)
(74) Representative: Powell, Timothy John

(57) **Abstract**

*Propionibacterium acnes*, *Propionibacterium avidum, Propionibacterium lymphophilum*, *Propionibacterium granulosum*, *Corynebacterium parvum*, and *Arachnia propionica* are effective in enhancing cell-mediated immunity when administered orally. These bacteria are capable of enhancing weight gain as well. The disclosed compositions and methods have been shown to work in pigs. They are expected to be effective in livestock, pets, and humans. The bacteria may be live, attenuated or killed. In addition, extracts or subunits may be used. The bacteria may be administered alone, or with food, water, or medications. Because the bacterial composition is stable under processing conditions, incorporation into commercially-available feed compositions is useful for administration to animals. Mixtures of the listed bacterial species are predicted to elicit the same results as a single species.

## Description

### Background of the Invention

The present invention relates to compositions and methods enhancing cell-mediated immunity and weight gain. More specifically, it relates to use of *Propionibacterium acnes*, *Propionibacterium avidum*, *Propionibacterium lymphophilum*, *Propionibacterium granulosum*, *Corynebacterium parvum*, and *Arachnia propionica* to enhance these effects.

Bacteria and their derivatives were among the first substances to be recognized as immunostimulators and are used as adjuvats in vaccines to boost the humoral immune response (e.g., complete Freund's adjuvat).

Bacteria have also been used as non-specific enhancers of the immune system to increase resistance and rejection of cancers, parasites, and infectious organisms. Bacterial preparations injected into animals and humans result in a number of immunological stimulatory effects which include increased macrophage activation and production, increased phagocytic cell activity, and natural killer cell activity, and increased production of cytokine proteins (e.g., interleukins and interferons). These can be broadly grouped as non-specific defense mechanisms also called cell-mediated immunity (CMI). Several products have been developed by pharmaceutical companies and licensed for use as immunostimulants or immunomodulators in animals and humans in the United States and Europe. To date, most products have been approved to treat specific diseases. These products are administered by intravenous, intraperitoneal, subcutaneous, or intralesional injection.

Other products have been used as immune modulators in livestock feed. Examples are purified β-glucan from yeast cell walls and a less purified yeast cell wall derivative containing mannan oligosaccharides. These type of products can have inconsistent activity, be unstable, and be costly.

The problem is that many bacteria do not pass through the gut wall when administered orally and are, therefore, unable to exert their potential stimulatory activities via this route. Providing immunostimulants orally can boost the intestinal immune system which can be a very important site of protection for the recipient.

Weight gain is one of the primary concerns of livestock producers. Any composition or method which conveniently and cost-effectively enhances weight gain in meat-producing animals is desired. Weight gain is also desired in humans, for example, after illness or in premature infants. Such weight gain may be therapeutic or merely cosmetic.

For the foregoing reasons, there is a need for an oral composition or oral dosing method which enhances a cell-mediated immunity response. In addition, there is a need for compositions and methods which enhance weight gain.

### Summary

It is an object of the present invention to provide an oral, bacterial composition which enhances cell-mediated immunity.

It is another object of the present invention to provide an oral, bacterial composition which enhances weight gain.

It is a further object of the present invention to provide an oral method which enhances cell-mediated immunity through the use of bacteria.

It is yet another object of the present invention to provide a oral method which enhances weight gain using bacteria.

These and other objects of the invention will become apparent upon reference to the following specification and claims.

We have discovered that *Propionibacterium acnes* (*P. acnes*) elicits a greater immune modulation response than other products on the market (e.g., glucan product). We have also discovered that this bacteria elicits increased weight gain.

It was desired to find a composition and/or a method which cost-effectively enhances CMI. In contrast to previous compositions and/or methods, we found oral administration of *P*. *acnes* to be an effective and more convenient method of stimulating CMI. These bacteria have been reported to be ineffective when administered orally so the results were unexpected. In addition, we found that oral administration of *P. acnes* enhances weight gain, which has never been recognized before with this group of bacteria.

The composition includes, as an active ingredient, bacteria selected from the group consisting of *Propionibacterium acnes*, *Propionibacterium avidum*, *Propionibacterium lymphophilum*, *Propionibacterium granulosum*, *Corynebacterium parvum*, and *Arachnia propionica*. Oral administration of this composition stimulates CMI when used in the proper concentration range. In addition, the composition improves weight gain. The method of enhancing cell-mediated immunity comprises the step of orally administering a composition comprising, as an active ingredient, bacteria selected from the group consisting of *P. acnes*, *P.* *Lymphophilum*, *P. granulosum*, *C. parvum*, *and A. propionica* at an appropriate concentration. This method also enhances weight gain.

The preferred embodiment is *P. acnes* administered as an additional ingredient in a commercially-available feed composition which is fed to starter pigs.

The composition/method can be used in a variety of forms and administered to a variety of recipients.

These and other features, aspects, and advantages of the present invention will become better understood with reference to the following description and appended claims.

### Detailed Description

The practice of the present invention will employ, unless otherwise indicated, conventional techniques of microbiology and immunology, which are within the skill of the art.

### A. Definitions

In describing the present invention, the following terms will be employed, and are intended to be defined as indicated below.

Animals, as used throughout the specification and claims, includes non-human animals, including vertebrates such as livestock (e.g., cattle, sheep, poultry, fish, and swine) and domestic animals (e.g., equines, dogs, and cats).

Pets includes domestic animals traditionally kept for pleasure such as canines and felines.

### B. Composition/Method

The bacteria used for practicing the present invention are the bacteria selected from the group consisting of *Propionibacterium acnes*, *Propionibacterium advidum***,** *Propionibacterium lymphophilum, Propionibacterium granulosum*, *Corynebacterium parvum* or *Arachnia propionica. P. acnes* will be the only bacteria referred to throughout the description because it is the preferred embodiment, although it should be clear that any of the bacterial species claimed may be substituted. The statements contained in this description should apply equally well to each of the claimed bacteria unless otherwise indicated. All of the bacteria are expected to have the same results due to their taxonomic similarity.

It is recognized that *C. parvum* is now thought to be synonymous with *P. acnes*, but it has been included in the list due to the use of the name that still exists in the art.

Whole bacteria have been used. Extracts or subunits thereof are anticipated to elicit similar effects because surface molecules (antigens) on the cell wall are the substances that trigger the CMI response, therefore, the whole cell is not always needed. The use of non-viable bacteria has been successful, therefore it is anticipated that live, attenuated, or killed bacteria will work equally well. This is anticipated because, as mentioned above, cell wall components are responsible for the activity. As long as these cell wall components are present, forms which reduce or retain the pathogenicity of the bacteria are only different in their potential side effects. The methods for producing these different forms can be any of those commonly used by a person skilled in the art. We anticipate that the bacteria need not be simply one species of the bacteria listed, but can be a mixture of the listed bacterial species as well. The bacterial species will work individually, therefore, they should work in a mixture since there should be no interaction between the bacteria which will interfere with the production of CMI or weight gain.

*Propionibacterium acnes* is known to be commercially available in forms such as a injectable solution (e.g., ImmunoRegulin® or EqStim® by Immuno Vet in Tampa, FL), but it may also be isolated and cultured by known, standard bacterial procedures or obtained from national culture collections (e.g., ATCC 6919). The bacteria used were obtained from ImmunoVet who produced them under U.S.D.A. Product Code 9350.00. The bacteria may be provided wet or dry. A dry form may be prepared by standard drying methods known to a person skilled in the art, such as freeze-drying or evaporation. For mixing with feed ingredients, it is preferred that the bacterial composition be dry, nonsticky, and finely-divided for blending with the feed composition. We believe a drying method using dry heat may yield a less sticky bacterial composition which can be more uniformly blended into the feed. The bacteria are stable under conditions similar to those present *in vivo*, in feed, and in feed processing. The bacterial composition is stable under acidic or basic conditions and after autoclaving and freezing. The bacteria also maintain their potency when mixed with typical fat and oil sources found in feed.

To assure administration at a desired level to animals, it is preferred to mix a dry bacterial composition with a dry feed composition to a predetermined concentration. Though in the preferred embodiment the bacteria are administered in a feed composition, there is no known interaction with the feed, and, therefore, any commercially-available feed that promotes the growth of the animal should work. In humans, the *P. acnes* may be added to or taken as a supplement to a well-balanced diet. The preferred concentration of *P. acnes* when incorporated into a commercially-available feed composition for her pigs is about 1 ppb to 1000 ppm. The optimum level in this range is thought to be about 1 ppm. The *P. acnes* can be incorporated by dry blending using standard mixing equipment. The *P. acnes* should be uniformly distributed throughout the feed. After mixing, if desired, the feed material may be further processed, such as by conversion to pellets. Most feed compositions for animals are composed of mixtures of feed ingredients. Though it is preferred that the bacteria be administered in food, it may be administered in any form, such as by itself, in bolus dosage form, pellets, capsules, or other oral form. We anticipate that the bacteria will work in any of these forms because of their stability under processing conditions, as indicated above. The bacteria may also be administered in water or with medications. However, control of the amount administered in water can be expected to be more difficult. If the bacteria is administered in water or other liquid form, it will mix more easily and uniformly if the bacterial formulation is also wet.

The feed composition used is one which enhances animal growth. For starter pigs, the feed composition is one comprising at least one starch-bearing substance, at least one protein-bearing substance, and at least one fat-bearing substance. A more specific feed composition comprises starch-bearing substances in the range of about 10-80% by weight, protein-bearing substances in the range of about 10-50% by weight, fat-containing substances in the range of about 2-20% by weight, and vitamin and trace minerals in the range of about 0.0001-5% by weight, possibly an antioxidant or antibiotics in the range of about 0.001-1% by weight. A preferred feed composition comprises starch-bearing substances in the range of about 10-40% by weight, protein-bearing substances in the range of about 15-30% by weight, fat-containing substances in the range of about 6-12% by weight, and vitamin and trace minerals in the range of about 0.0001-5% by weight. The starch-bearing substance is generally a grain product selected from the group consisting of corn, soybean, wheat, sorghum, barley, oat and mixtures thereof. Examples include ground corn, corn flour, oat groats, wheat flour, oat flour, soybean flour, wheat starch, wheat middlings, soybean meal, corn grit, oat meal, and mixtures thereof. The preferred starch-bearing substance is selected from the group consisting of oat flour, oat groats, ground corn, wheat middlings, soybean meal, and mixtures thereof. The protein-bearing substance is generally a protein-bearing substance selected from the group consisting of fish meal, dried whey, soybean meal, soybean protein concentrate, soy flour, soybean protein isolate, blood meal, plasma protein, dried skim milk, whey protein concentrate, canola meal, corn gluten meal, wheat gluten meal, sunflower meal, and mixtures thereof. The preferred protein-bearing substance is selected from the group consisting of fish meal, dried whey, blood meal, plasma protein, and soybean meal. The fat-bearing substance is generally a fat-bearing substance selected from the group consisting of lard, tallow, soybean oil, lecithin, coconut oil, whey-fat blend, poultry fat, corn oil, and mixtures thereof. The preferred fat-bearing substance is selected from the group consisting of soybean oil, coconut oil, and lard. The vitamins are selected from the group consisting of A, D, E, K, riboflavin, pantothenic acid, niacin, vitamin B12, folic acid, pyridoxine, biotin, vitamin C, thiamin and mixtures thereof. The trace minerals are selected from the group consisting of copper, zinc, iodine, selenium, manganese, iron, cobalt, compounds thereof, or mixtures thereof. The antioxidants are selected from the group consisting of ethoxyquin, BHT, BHA, vitamin E, ascorbic acid, and mixtures thereof. The antibiotics are selected from the group consisting of chlorotetracycline, sulfathiazole, penicillin, carbadox, apramycin, oxytetracycline, sulfamethazine, neomycin, tylosin, lincomycin, and mixtures thereof. The most preferred feed composition used with *P. acnes* for starter pigs comprises 0.001-70% ground corn, 0.001 to 30% oat flour, 0.001 to 30% oat groats, 0.001 to 15% fish meal, 0.001 to 40% dried whey, 0.001 to 40% wheat middlings, 0.001 to 30% whey-fat blend, 0.001 to 5% animal or vegetable fat, 0.001 to 10% plasma protein, 0.001 to 4% calcium carbonate, 0.001 to 5% dicalcium phosphate, 0.001 to 4% acidifier, 0.001 to 1% flavor or aroma, 0.001 to 3% antibiotic medication, 0.001 to 1% vitamin-trace mineral mix, 0.001 to 1% lysine HCl, 0.001 to 0.2% DL-methionine, 0.001 to 0.1% L-tryptophan, 0.001 to 0.2% L-threonine, and 0.001 to 0.5% antioxidant, wherein the % is weight %.

The previously stated preferred concentration of *P. acnes* in feed relates primarily to feeds which are formulated to comprise substantially the total ration of the animal. If feed supplements or feed concentrates are employed as the vehicle for administering the bacteria, different concentrations may be required. The concentrations can be related to either the total diet of the animal or to the body weight of the animals being fed.

Indirect evidence of the involvement of the immune system, and accordingly induction of CMI, comes from small rises in body temperature after oral exposure to *P. acnes.* Interleukin-1, secreted by activated macrophages, is known to induce fever. Thus, a short-term rise in body temperature is indicative of cytokine release. The activity of *P. acnes* at low doses also suggests that there are indirect effects in the body. The low dose and the fact that non-viable or dead *P. acnes* is effective also rules out an effect brought about by replication or invasion of the organism in the animal. Cell-mediated immunity can be expected to produce may different effects. We anticipate that these effects will include uniform gain of weight, reduction of mortality and morbidity, reduction of infection, short-term protection against microbial (viral, bacterial, protozoal and fungal) infections, increase in feed intake, improvement in feed efficiency, increased carcass lean content, potentiation of vaccines (adjuvant effect), production of anti-inflammatory effects, and a general increase in health status leading to improved reproduction efficiency.

The weight gain may be a result of many possible mechanisms, including CMI.

As previously stated, the composition/method of this invention is expected to be effective for use in cattle, sheep, poultry, and swine, as well as domestic animals such as horses, dogs, and cats because we believe pigs to be an adequate model for these types of animals. In addition, we anticipate the composition and method will be effective when used on poultry and aquatic animals. Oral application of immunostimulants to achieve weight gain in fish is well-documented in the literature. (See, for example, Stosick, et al., *Medycyna Weterynaryjna* 52: 467-469 (1996).) The preferred recipient of the *P. acnes* is swine, especially her pigs. Since pigs are generally good models for humans physiologically, anatomically and immunologically (e.g., porcine insulin), the composition is anticipated to be effective when administered to humans. The concentration of the bacteria required to achieve CMI or weight gain will vary from species to species of the recipient. The appropriate concentration for a particular species of bacteria or recipient can be determined by techniques such as illustrated in the examples.

Although the composition and method have been described with respect to a preferred embodiment thereof, it is to be also understood that it is not to be so limited since changes and modifications can be made therein which are within the full intended scope of this invention as defined by the appended claims.

The method/composition of this invention is further illustrated by the following experimental examples.

### Example 1

### Stability of P. acnes under various conditions

### Acid/Base and Drying Stability

The *P. acnes* solution was subjected to various conditions to further evaluate its stability under extreme conditions. An aliquot of the solution was adjusted to a pH of 2 with dilute HCl for 1 hour, then adjusted to pH of 7 with dilute NaOH. The material was then potency tested in mice. Another aliquot of the solution was adjusted to a pH of 8 for 1 hour with dilute NaOH, then readjusted to a pH of 7 with dilute HCl. This sample was also evaluated via mouse potency. Another aliquot of the solution was placed in a sterile open petri dish under a MBSC hood and allowed to dry for 24 hours. The material was reconstituted to its normal volume with sterile saline and tested for potency via mice. The potency test consists of injecting intraperitoneally *P. acnes* in a saline solution into mice, and 72 hours later 0.5 mL *Salmonella typhimurium* at a dilution of 1:450,000 is injected intraperitoneally. The potency test, as designed, requires that 80% of the positive controls die and that at least 70% of the *P. acnes*-treated mice survive. Results are as shown in Table 1.

**TABLE 1**

| Treatment | # of Mice Tested | # Surviving | % Survival |
|---|---|---|---|
| Controls 0 mg/mouse | 20 | 2 | 10% |
| Acid Treatment 0.05 mg/mouse | 20 | 16 | 80% |
| Base Treatment 0.05 mg/mouse | 20 | 19 | 95% |
| Drying 0.05 mg/mouse | 20 | 18 | 90% |

### Freeze-Thaw Study

A freeze-thaw study was conducted on the *P. acnes* product. The *P. acnes* material used for the freeze-thaw test was a solution for which a standard potency test had been conducted, showing that the product protected 20 out of 20 mice and only 2 out of 20 of the negative controls.

Five by five mL bottles were frozen at -15 °C (±5 °C) for approximately 8 hours and subsequently thawed at room temperature for approximately 15 hours, three times each. Mice were inoculated with a composite mixture of the 5 freeze-thawed bottles, challenged 3 days later, and then results recorded 14 days later.

All of the treated mice inoculated with the freeze-thawed material survived (20/20), while only 2/20 of the negative controls for this test survived showing that the *P. acnes* product did not lose any of its effectiveness after being frozen and thawed several times at the specified temperatures.

### Autoclaving

Aliquots of *P. acnes* solution were subjected to steam sterilization at 121 °C for 15 minutes at 15 psi. This was accomplished to prepare inactive placebo for studies. When tested via mouse potency tests to confirm antigen degradation, the product was still found to provide more than 70% survival rates.

### Presence of Feed Ingredient Fat and Oil Sources

A potency test (as described under Acid/Base and Drying Stability above) was performed in order to show the stability of *P. acnes* in the presence of typical fat and oil sources found in feed. Twenty mice were tested. The results are shown in Table 2.

**TABLE 2**

| 14-Day Mouse Test | Positive Control | *P. acnes* | *P. acnes +* oil + emulsifier | *P. acnes* + emulsifier |
|---|---|---|---|---|
| # survivors | 4 | 19 | 17 | 17 |
| % survivors | 20% | 95% | 85% | 85% |

### Example 2

### Non-specific Stimulation of Pig Immunity

This example shows the rise in temperature found in pigs when *P. acnes* is administered orally which is indicative of a immunological response.

### Materials and Methods

A randomized, complete-block experiment was performed for 5 different levels of *P. acnes* for 7 days. There were 4 to 5 weaned pigs in each pen. Each dietary treatment was represented by 5 weight replicates with the dietary program designed to optimize growth. The *P. acnes* was fed for the first 7 days only. The rectal temperature of one random pig per pen was measured on days 0, 1, and 7 of the trial to determine if *P. acnes* caused a rise in body temperature. A temporary increase in temperature can be used as an indirect measure of *P. acnes* on the immune system through stimulating the macrophages creating a release of cytokines which cause a rise in temperature.

### Results

Table 3 summarizes the results of the experiment. The rectal temperature of pigs at the start of the trial ranged between 101.6 and 102.4 °F (Day 0 avg. =102.0) and was used as a covariate for the other temperature data. There was a 0.6 and 0.9 °F decrease in rectal temperature on day 1 for pigs fed 1.25 and 10 ppm *P. acnes* as well as a 0.3 °F increase on day 1 for pigs fed 2.5 ppm *P. acnes.* On day 7 of the trial, pigs which had been fed 0.625 and 5 ppm *P. acnes* had a sighificant (P≤.10) temperature increase of 0.9 to 1 °F relative to day 0. The activity of *P. acnes* at low doses of 1 to 10 ppm also suggested that there are indirect effects in the body. The low dose and the fact that the *P. acnes* was non-viable or dead also rules out any effect due to replication or invasion in the animal.

**TABLE 3**

| Influence of Level of *Propionibacterium acnes* on the rectal temperatures of starter pigs. | | | | | | |
|---|---|---|---|---|---|---|
| | Level of *Propionibacterium acnes* fed for 7 days, ppm | | | | | |
| | 0 | 0.625 | 1.25 | 2.5 | 5 | 10 |
| Treatment | 1 | 2 | 3 | 4 | 5 | 6 |
| **Rectal Temperatures, °F** | | | | | | |
| Day 1 | 101.92 | 101.90 | 101.43 | 102.30 | 101.99 | 101.12 |
| Day 7 | 102.16 | 103.05 | 102.51 | 102.25 | 102.90 | 101.96 |

| **Temperature change, °F** | | | | | | |
|---|---|---|---|---|---|---|
| Day 1 | -0.11 | -0.13 | -0.59 | 0.27 | -0.04 | -0.91 |
| Day 7 | 0.13 | 1.03 | 0.48 | 0.23 | 0.87 | -0.07 |

### Example 3

### Effect of P. acnes level on weight gain

This example shows the weight gain induced by addition of *P. acnes* to pig diets.

### Materials and Methods

The pigs in the experiment of Example 2 were weighed at 0, 7, 13, and 33 days. There was also a set of pigs fed an existing market product (MacroGard®, purified β-glucan from yeast cell walls) at its manufacturer recommended dosage.

### Results

Table 4 summarizes the results of the experiment. There were slight increases in pig weights, average daily gain, and daily feed intake during the first week of the trial for all levels of *P. acnes* fed, with the 0.625 and 10 ppm levels being slightly higher than the 2.5 and 5 ppm levels. MacroGard® at 0.05% also increased performance more than the *P. acnes* added to the diet during the first week of the trial (P<.10). From days 7 to 13, levels of 1.25 and 2.5 ppm increased pig weights and average daily gain, whereas there were slight depressions in daily gain with 5 and 10 ppm. The performance of pigs from 7 to 13 days postweaning was higher (P<.10) from pigs fed 0.05% MacroGard® relative to any of the *P. acnes* levels.

From 13 to 33 days, there were improvements in average daily gain (P<.10) and feed intake (P<.05) from pigs which were fed 1.25 ppm *P. acnes* relative to control pigs. There was also a tendency (P>.10) for an improvement in average daily gain and feed intake from 13 to 33 days postweaning for pigs fed 10 ppm *P. acnes.* Performance of pigs fed 0.025% MacroGard® was slightly lower from day 13 to day 33 of the trial relative to pigs fed either 1.25 or 10 ppm *P.* *acnes*. Final pig weight at day 33 was very similar between pigs fed MacroGard® throughout the trial or pigs fed 1.25 ppm *P. acnes*.

There were significant improvements in 33-day weights and overall daily gain for pigs fed the 0.05/0.025% MacroGard® ration sequence relative to control pigs. Average daily gain during the overall trial was highest for pigs fed 1.25, 2.5, or 10 ppm of *P. acnes*. Overall daily feed intake was increased for pigs which had been fed 1.25 or 2.5 ppm as well as pigs fed MacroGard®. There were no clear differences in the overall feed efficiency of pigs with respect to level of *P. acnes* or MacroGard® added to the diet during the 33-day trial.

The *P. acnes* produced similar growth responses at 50 to 100-fold lower concentrations (1 to 10 ppm) when fed for 7 days than the treatment with a existing market product fed for the entire 33 day test.

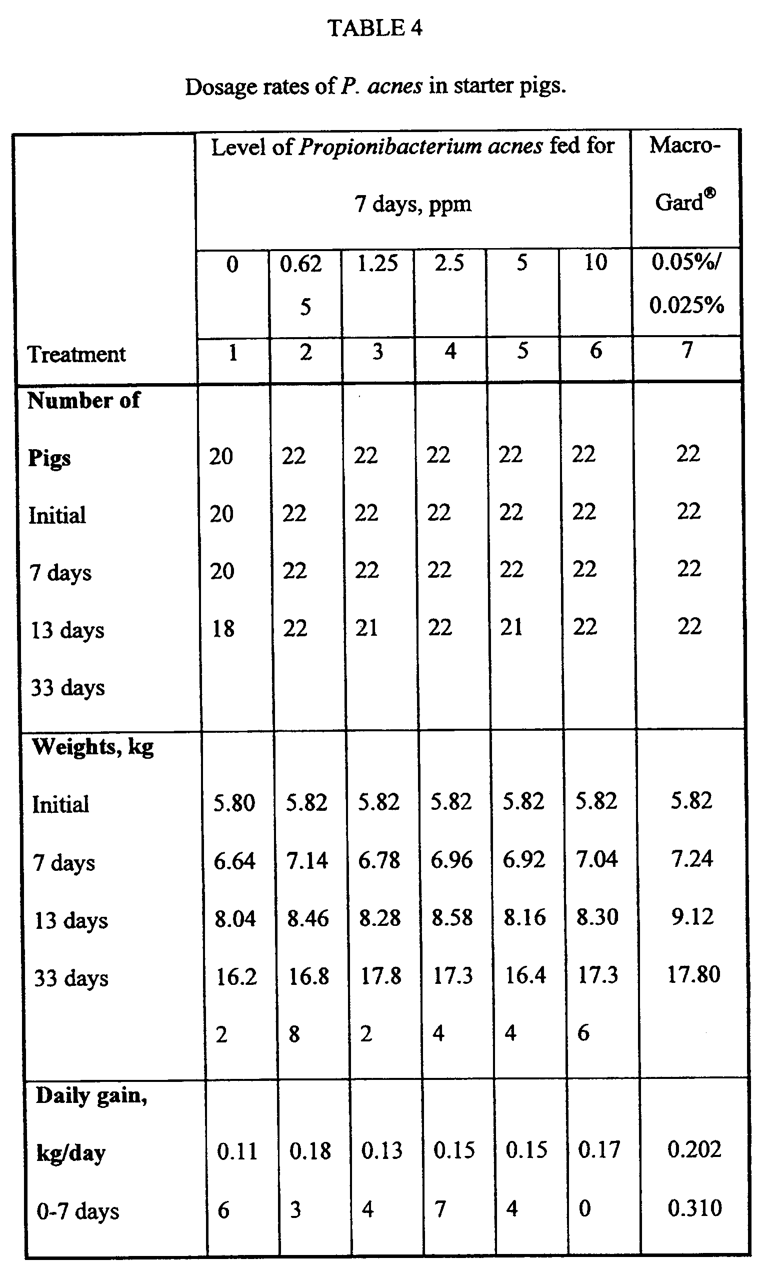

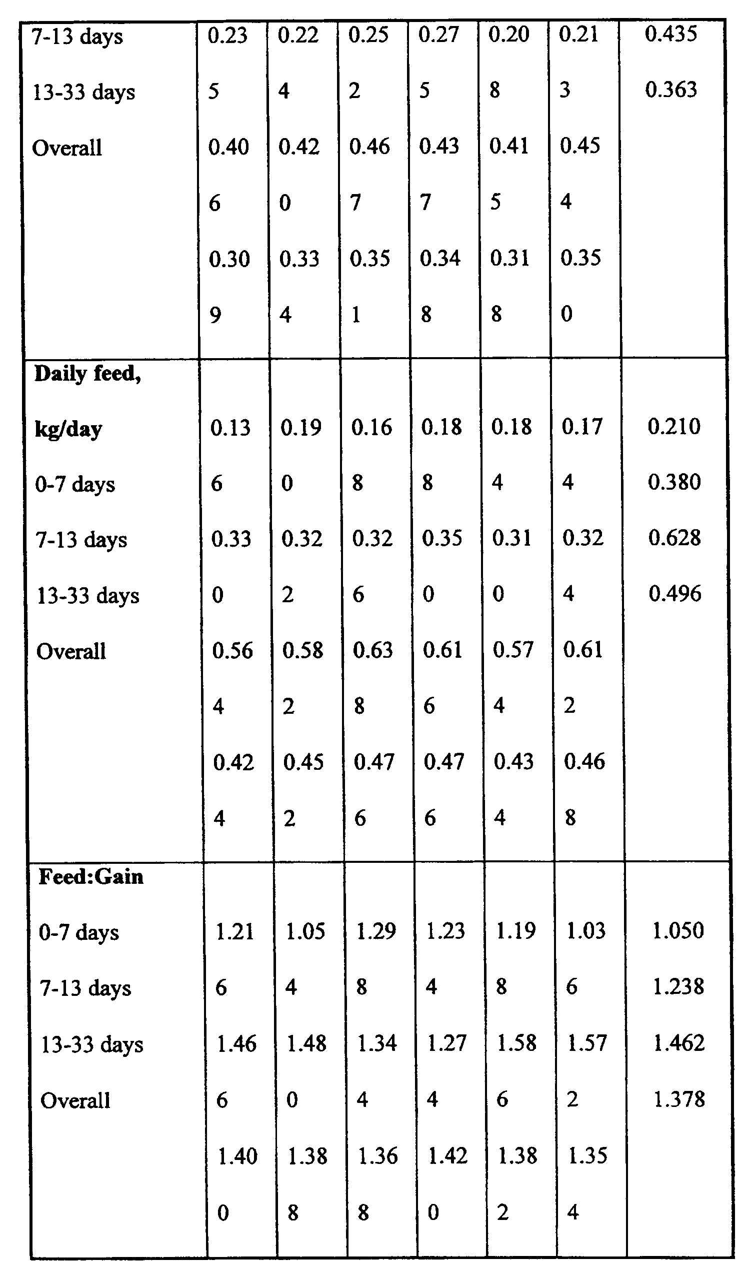

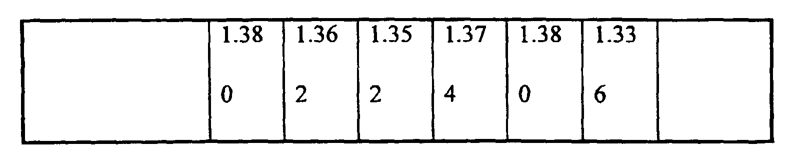

### Example 4

### Effects on Weight Gain in Pigs of P. acnes level and length of feeding

This example shows the effect of length of feeding time and effect of concentration on weight gain in pigs fed *P. acnes*.

### Materials and Methods

Six replicate pens of pigs (initial weight=6.35 kg) were fed a control diet, 1, 10, or 100 ppm *P. acnes* for 7 days or 1 ppm for 13 days.

### Results

Table 5 summarizes the results of this experiment. Pigs fed 1, 10, or 100 ppm *P. acnes* for 7 days had increased rate of gain (P<.05) from 13 to 33 days. Pigs fed 1 or 10 ppm *P. acnes* for 7 days had greater (P<.10) gain and body weight for the entire experiment.

From 7 to 13 days there were no treatment effects (P>.10) on gain, feed intake or feed efficiency.

From 13 to 33 days of the experiment, the most dramatic effects of *P. acnes* were observed. Feeding *P. acnes* for 7 days at 1, 10 or 100 ppm increased (P<.05) gain to a similar extent relative to pigs fed the control diet. A portion of this response was due to increased feed intake; however, there were improvements in feed efficiency also. The greatest improvement in feed efficiency was noted for pigs fed 100 ppm *P. acnes* for 7 days. Pigs fed 1 ppm *P. acne*s for 7 days were more efficient (P<.05) than pigs fed the control diet; however, pigs fed 10 ppm for 7 days had similar (P>.10) feed efficiency to pigs fed the control diet.

For the overall 33 day experimental period, the effects noted from 13 to 33 carried through for the most part. Specifically, pigs fed either 1 or 10 ppm for 7 days had a greater rate of gain than pigs fed the control diet. This response was primarily due to increased feed intake; however, as noted for days 13 to 33, improvements in feed efficiency were also noted.

The improvement in growth of pigs fed 1 or 10 ppm for 7 days resulted in pigs fed those treatments being in excess of 1 kg heavier (P<.05) than pigs fed the control diet at the end of the experiment. Feeding 100 ppm for 7 days numerically decreased the rate of gain from days 0 to 7, did not change gain from 7 to 13, and increased gain from 13 to 33. The summation of these growth responses resulted in pigs fed 100 ppm for 7 days being numerically heavier than the control pigs at the end of the experiment.

Feeding 1 ppm for 13 days did not result in significant improvements in performance, although the trend was positive. The growth response from feeding 100 ppm for 7 days was not different from pigs fed 1 or 10 ppm for 7 days. Based on the data collected to this date, it seems that 1 ppm fed for 7 days is an optimal effective dose.

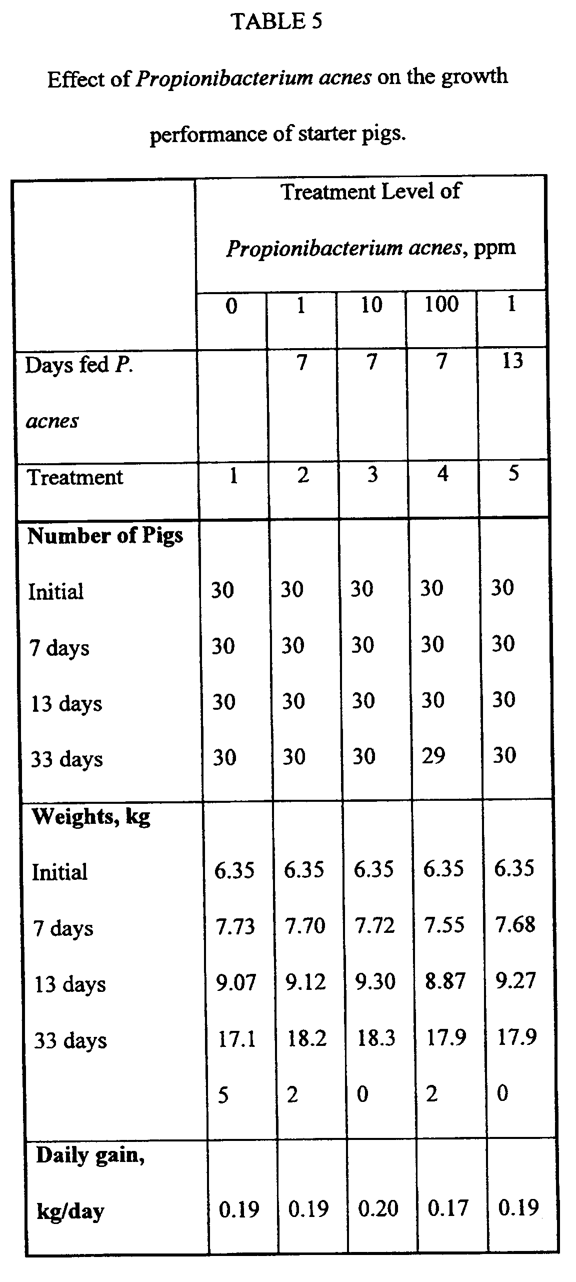

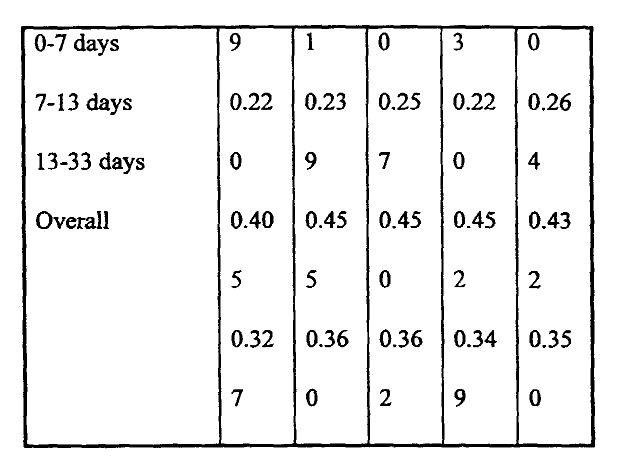

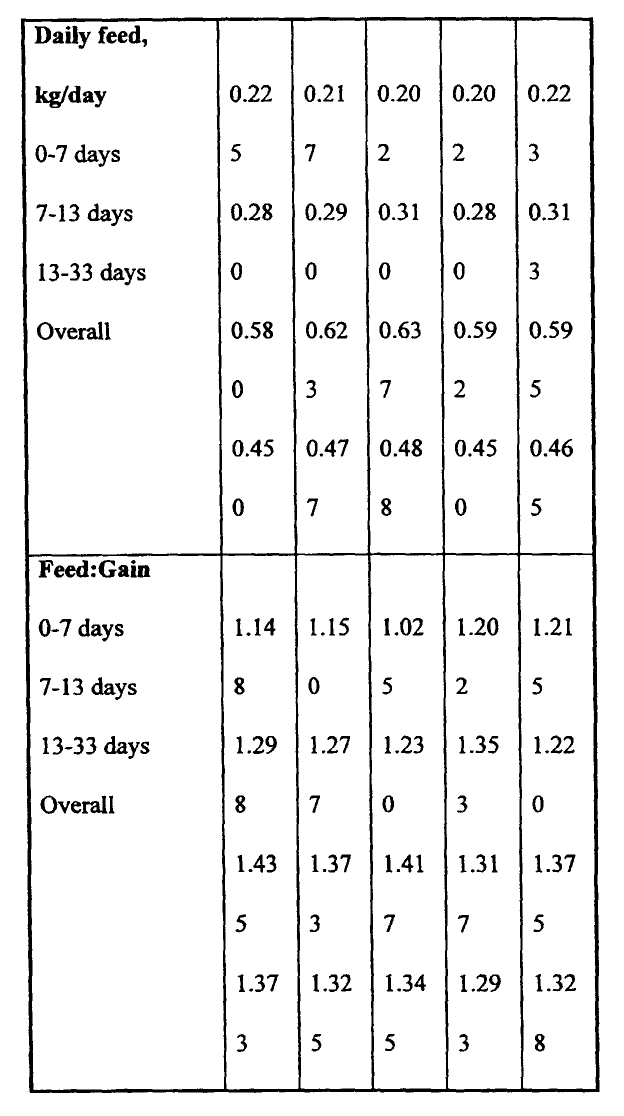

The foregoing description and examples merely explain and illustrate the invention and the invention is not limited thereto, except insofar as the claims are so limited, as those skilled in the art who have the disclosure before them will be able to make modifications and variations therein without departing from the scope of the invention. For example, *P. acnes* can be incorporated into a dry carbohydrate feed carrier or included with an oral iron supplement. Species of the genus Propionibacterium, other than those listed in this disclosure, may work in the disclosed compositions and methods. The composition in combination with β-glucan or Acemannan may have a synergistic effect. A combination of the composition with β-glucan may produce a cost benefit by reducing the amount required. The compositions/methods may work in combination with adjuvants or probiotics.

## Claims

1. An oral composition comprising, as an active ingredient, bacteria or extracts of the bacteria, the bacteria being selected from the group consisting of *Propionibacterium acnes*, *Propionibacterium avidum*, *Propionibacterium lymphophilum*, *Propionibacterium granulosum*, *Corynebacterium parvum* and *Arachnia propionica* administered orally at a concentration which enhances cell-mediated immunity or weight gain or both.

2. The composition of Claim 1 wherein the bacteria are whole, nonviable bacteria.

3. The composition of Claim 1 wherein the bacteria are live.

4. The composition of Claim 1 comprised of extracts of the bacteria.

5. The composition of Claim 1 for administration to animals.

6. The composition of Claim 5 wherein the animals are selected from the group including humans, cattle, swine, poultry, fish, rabbits, sheep, pets and shellfish.

7. The composition of Claim 1 administered to humans.

8. The composition of Claim 1 or 2 administered with food, wherein said food is a feed composition which enhances animal growth, said feed composition comprising at least one starch-bearing substance, at least one protein-bearing substance, and at least one fat-bearing substance.

9. A method of enhancing cell-mediated immunity or weight gain which comprises the step of orally administering a composition according to any one of Claims 1 to 8.
